(19)

Europäisches
Patentamt

European
Patent Office

Office européen
des brevets

(11) **EP 3 754 329 B1**

(12) **EUROPÄISCHE PATENTSCHRIFT**

(45) Veröffentlichungstag und Bekanntmachung des
Hinweises auf die Patenterteilung:
**26.07.2023   Patentblatt 2023/30**

(21) Anmeldenummer: **19181581.0**

(22) Anmeldetag: **21.06.2019**

(51) Internationale Patentklassifikation (IPC):
**G01N 27/12** (2006.01)     **G01N 33/00** (2006.01)

(52) Gemeinsame Patentklassifikation (CPC):
**G01N 27/12; G01N 33/005**

(54) **WASSERSTOFFSENSOR UND VERFAHREN ZU DESSEN HERSTELLUNG, MESSVORRICHTUNG UND VERFAHREN ZUM MESSEN EINER WASSERSTOFFKONZENTRATION**

HYDROGEN SENSOR AND METHOD FOR PRODUCING THE SAME, MEASURING DEVICE AND METHOD FOR MEASURING HYDROGEN CONCENTRATION

CAPTEUR D'HYDROGÈNE ET SON PROCÉDÉ DE FABRICATION, DISPOSITIF DE MESURE ET PROCÉDÉ DE MESURE DUNE CONCENTRATION D'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.12.2020   Patentblatt 2020/52**

(73) Patentinhaber: **Materion GmbH**
**23966 Wismar (DE)**

(72) Erfinder: **WIENECKE, Marion**
**23966 Wismar (DE)**

(74) Vertreter: **Seemann & Partner Patentanwälte mbB**
**Raboisen 6**
**20095 Hamburg (DE)**

(56) Entgegenhaltungen:
CN-A- 108 872 314     DE-A1- 10 011 164
DE-B4- 10 011 164     US-A- 3 479 257

• Hongchuan Jiang ET AL: "Low Concentration Response Hydrogen Sensors Based on Wheatstone Bridge", , 25. Februar 2019 (2019-02-25), XP055650599, DOI: 10.20944/preprints201902.0228.v1 Gefunden im Internet: URL:https://www.preprints.org/manuscript/2 01902.0228/v1 [gefunden am 2019-12-09]

**Beschreibung**

Die Erfindung betrifft eine Messvorrichtung zur Messung einer Wasserstoffkonzentration in einer Umgebung, umfassend eine Messeinheit und einen Wasserstoffsensor. Schließlich betrifft die Erfindung ein Verfahren zum Messen einer Wasserstoffkonzentration in einer Umgebung.

Wasserstoffsensoren, mit denen Wasserstoffkonzentrationen in Gasen, beispielsweise in Luft, gemessen werden können, sind in verschiedenen Ausführungen bekannt. Die Vielzahl der marktverfügbaren Wasserstoffsensoren basiert auf einigen wenigen physikalischen Messprinzipien. Eines dieser physikalischen Prinzipien ist die Fähigkeit verschiedener Materialien Wasserstoff zu absorbieren. In Abhängigkeit der in dem als Sensor eingesetzten Material gelösten Menge an Wasserstoff verändert sich eine oder mehrere von dessen messbaren physikalischen Eigenschaften.

Ein Wasserstoffsensor, bei dem eine Veränderung der optischen Eigenschaften des Sensormaterials ausgenutzt wird, ist aus DE 10 2006 018 767 A1 bekannt. Bei dem aus diesem Dokument bekannten Sensor wird ein Sensormedium mit elektromagnetischer Strahlung beleuchtet und mittels eines Detektors wird ein Transmissionskoeffizient des Sensormediums gemessen. Als Sensormedium kommt also ein Material zum Einsatz, dessen Transmissionskoeffizienten in Abhängigkeit der Wasserstoffkonzentration in einer Messumgebung des Sensormediums variieren. Der gemessene Transmissionskoeffizient wird als Maß für die Wasserstoffkonzentration in der Umgebung herangezogen.

Aus US 7,791,150 B1 geht ein Wasserstoffsensor hervor, bei dem auf einem Siliziumwafer, der auf seiner Oberseite eine thermisch erzeugte Oxidschicht aufweist, zunächst eine Elektrodenkonfiguration strukturiert aufgebracht wird. Auf diese wird ein wasserstoffsensitiver Halbleiteroxidfilm ($In_2O_3$ dotiert mit $SnO_2$) aufgebracht. Dieser wasserstoffsensitive Film wird beispielsweise in einem Sol-Gel-Verfahren aufgebracht. Außerdem wird eine Oberseite dieses Films mit einer wasserstoffselektiven Schicht versehen. Es wird eine Änderung der elektrischen Eigenschaften des Halbleiteroxidfilms als Maß für die Wasserstoffkonzentration herangezogen.

Ein weiterer Wasserstoffsensor ist aus KR 20180074100 bekannt. Es wird ein Schichtsystem bestehend aus einer Polymerfolie, die als Substrat dient, einer haftvermittelnden Schicht oder Lage und einer auf dieser aufgebrachten Palladiumschicht bereitgestellt. Wird die Palladiumschicht einer wasserstoffhaltigen Umgebung ausgesetzt, so verändert die Palladiumschicht ihr Volumen. Diese Volumenänderung führt dazu, dass sich in dem oben beschriebenen Schichtsystem mechanische Spannungen aufbauen. Das Schichtsystem ist als Biegebalken aufgebaut. Diese mechanischen Spannungen führen wiederum dazu, dass sich der Biegebalken in Abhängigkeit der Wasserstoffkonzentration mehr oder weniger stark krümmt.

Aus CN 108872314 ist ein Wasserstoffsensor bekannt, bei dem auf Zinkoxid (ZnO) als Basismaterial eine Schicht aus Palladium aufgebracht wird. Der bereits zuvor beschriebene Effekt, dass Palladium bei Aufnahme von Wasserstoff eine Volumenänderung erfährt, wird bei diesem Sensor dazu ausgenutzt, eine mechanische Kraft auf das Zinkoxid auszuüben. Da das Zinkoxid einen piezoelektrischen Effekt zeigt, kann auf diese Weise ein passiver Sensor bereitgestellt werden. Die piezoelektrische Spannung am Zinkoxid ist ein direktes Maß für die Wasserstoffkonzentration, der der Palladiumfilm ausgesetzt ist.

Aus der Veröffentlichung Hongchuan Jiang et al.: "Low Concentration Response Hydrogen Sensors Based on Wheatstone Bridge", preprint, 2019, ist ein Wasserstoffsensor bekannt, bei dem auf ein Siliziumsubstrat strukturierte Leiterbahnen aufgebracht werden. Die Leiterbahnen bestehen aus PdNi. Das PdNi verändert in Abhängigkeit der Wasserstoffkonzentration, die aus der Umgebung in dem Material gelöst wird, seinen elektrischen Widerstand, so dass durch Messung des Widerstands der Leiterbahnen ein Wasserstoffgehalt messbar ist.

Die schaltbaren physikalischen Eigenschaften von Palladium beruhen auf dem Effekt der atomaren Einlagerung von Wasserstoff auf Zwischengitterplätzen. In Abhängigkeit der Wasserstoffkonzentration, d.h. der im Metallgitter gelösten Menge an Wasserstoff, verändert sich die Gitterkonstante des Palladiums, die mit der Volumenausdehnung des Materials einhergeht. Der Effekt ist reversibel.

Es ist eine Aufgabe der Erfindung, einen verbesserten Wasserstoffsensor, eine verbesserte Messvorrichtung sowie ein verbessertes Verfahren zum Messen einer Wasserstoffkonzentration und ein verbessertes Verfahren zum Herstellen eines solchen Wasserstoffsensors anzugeben.

Die Aufgabe wird gelöst durch eine Messvorrichtung zur Messung einer Wasserstoffkonzentration in einer Umgebung, umfassend eine Messeinheit und einen Wasserstoffsensor zur Messung einer Wasserstoffkonzentration in einer Umgebung, wobei der Wasserstoffsensor ein Substrat umfasst, auf dem in einem mit der Umgebung kommunizierenden Sensorbereich ein Wasserstoff absorbierendes Sensormedium als dünne Schicht aufgebracht ist, wobei das Sensormedium sein Volumen in Abhängigkeit einer in dem Sensormedium vorhandenen Wasserstoffkonzentration verändert, und diese Variation des Volumens eine Variation einer von dem Sensormedium in das Substrat eingebrachten mechanischen Spannung bewirkt, wobei das Substrat zumindest im Sensorbereich ein piezoresistiver Halbleiter ist, wobei das Sensormedium mit der Umgebung kommuniziert, wobei die Messvorrichtung dadurch weitergebildet ist, dass die Messeinheit dazu eingerichtet ist, einen ohmschen Widerstand des Substrats zu messen und aus dem Wert des gemessenen ohmschen Widerstands eine Wasserstoffkonzentration in der Umgebung zu bestimmen.

Mit Hilfe des erfindungsgemäßen Wasserstoffsensors kann ein gro-βer Messbereich von Wasserstoffkonzentrationen gemessen werden. Außerdem weist der Sensor keine Querempfindlichkeiten zu anderen Gasen auf und zeigt eine schnelle Reaktionszeit. Der Sensor nutzt die Volumenänderung des Sensormediums bei Wasserstoffabsorptionen aus. Das Sensormedium bringt in Abhängigkeit von der Wasserstoffkonzentration unterschiedlich große mechanische Spannungen in das Substrat ein. Indem außerdem die piezoresistiven Eigenschaften des Halbleiters ausgenutzt werden, können diese mechanischen Spannungen und somit die Waserstoffkonzentration einfach und hochpräzise gemessen werden. Vorteilhaft können die mechanischen Spannungen im Sensorbereich des piezoresistiven Halbleiters elektrisch gemessen werden, beispielsweise indem auf dem Halbleiter Widerstandsstrukturen in Form einer Wheatstonebrücke vorgesehen werden.

Gemäß einer Ausführungsform ist vorgesehen, dass das Sensormedium ein Metall oder eine Metalllegierung ist.

Gemäß einer weiteren Ausführungsform ist vorgesehen, dass der piezoresistive Halbleiter Silizium ist. Mit anderen Worten besteht das Substrat des Wasserstoffsensors zumindest im Sensorbereich aus Silizium.

Ferner ist insbesondere vorgesehen, dass das Sensormedium aus Palladium, Yttrium, Scandium, einem Lanthanid, einem Actionid, Wolframoxid und/oder Vanadiumoxid ist.

Gemäß einer weiteren Ausführungsform ist vorgesehen, dass das Sensormedium eine Palladiumlegierung oder eine Mischung, Legierung oder Verbindung aus mehreren der folgenden Materialien ist: Palladium, Yttrium, Scandium, einem Lanthanid, einem Actionid, Wolframoxid, Vanadiumoxid.

Gemäß einer weiteren vorteilhaften Ausführungsform ist vorgesehen, dass das Sensormedium eine Legierung aus Palladium und Gold ($Pd_xAu_y$-Legierung) oder eine Legierung aus Palladium und Nickel ($Pd_x$-$Ni_y$-Legierung ist, wobei insbesondere der Anteil von Gold oder Nickel zwischen 0,5at% und 50at% liegt ($Pd_{99,5}Au_{0,5}$-Legierung bis $Pd_{50}Au_{50}$-Legierung oder $Pd_{99,5}Ni_{0,5}$-Legierung bis $Pd_{50}Ni_{50}$-Legierung)

Das Sensormedium absorbiert Wasserstoff, wobei die Wasserstoffatome im atomaren Gitter des Sensormediums auf Zwischengitterplätzen eingelagert werden. Dies gilt insbesondere für die Verwendung von Palladium oder einer Palladiumlegierung als Sensormedium, aber auch für alle anderen genannten Materialien.

Bei der Einlagerung von Wasserstoffatomen im atomaren Gitter treten verschiedene an sich bekannte physikalische Effekte auf. Einer dieser Effekte, welcher von dem erfindungsgemäßen Wasserstoffsensor jedoch gezielt nicht ausgenutzt wird, ist der Übergang von der α-Phase in die β-Phase. Dies gilt, wenn Palladium oder eine Palladiumlegierung als Sensormedium verwendet wird. Während die α-Phase metallisch opak ist, ist die β-Phase des Palladiums transparent oder zumindest transparenter als die α-Phase. Dieser Phasenübergang kann ausgenutzt werden, um optisch eine Wasserstoffkonzentration in dem als Sensormedium eingesetzten Palladium zu messen. Dies hat jedoch den technischen Nachteil, dass der Übergang von der α-Phase zur β-Phase stattfinden muss, bevor ein Messsignal zur Verfügung steht. Gleiches gilt für eine Messung, welche auf dem physikalischen Effekt beruht, dass die α-Phase metallisch leitfähig und die β-Phase halbleitend ist. Auch bei einem Sensor, der diesen Effekt ausnutzt, muss der Phasenübergang stattfinden. Beispielsweise liegt die α-Phase im Palladium bis zu einer Wasserstoffkonzentration von 1,68at% vor. Dies bedeutet, dass geringe Wasserstoffkonzentrationen mit einem solchen Sensor, welcher den oben beschriebenen Phasenübergang ausnutzt, nicht oder nur schlecht gemessen werden können.

Die oben skizzierten technischen Nachteile werden bei dem erfindungsgemäßen Sensor vorteilhaft beseitigt. Noch während sich beispielsweise das Palladium in der α-Phase befindet, also bei geringen Wasserstoffkonzentrationen, tritt eine Veränderung des Volumens des Sensormediums durch die auf den Zwischengitterplätzen eingelagerten Wasserstoffatome auf. Diese mit einer Veränderung der Gitterkonstanten einhergehende Volumenänderung führt dazu, dass die Sensorschicht eine mechanische Spannung auf das Substrat ausübt. Diese Spannung kann anhand des piezoresistiven Effekts im Substrat gemessen werden. Somit ist es mit dem erfindungsgemäßen Sensor möglich, auch geringe Wasserstoffkonzentrationen mit hoher Genauigkeit zu erfassen.

Die oben erläuterten physikalischen und technischen Effekte werden lediglich beispielhaft für Palladium erläutert. Sie treten auch bei den übrigen genannten Materialien mehr oder weniger stark auf.

Der Wasserstoffsensor ist vorteilhaft dadurch fortgebildet, dass das als dünne Schicht ausgebildete Sensormedium eine Schichtdicke aufweist, die geringer als 500 nm ist, die insbesondere zwischen 5 nm und 100 nm und ferner insbesondere zwischen 5 nm und 20 nm liegt.

Die zuvor genannten Schichtdicken haben sich in der Praxis als vorteilhaft herausgestellt. Einerseits kann mit einer Sensorschicht, die eine Schichtdicke in den angegebenen Bereichen aufweist, eine ausreichend große mechanische Spannung erzeugt werden, so dass ein in dem Sensorbereich des Substrats messbarer Effekt auftritt. Andererseits ist der Aufwand zur Herstellung der Schicht überschaubar. Es findet keine Delamination der aufgebrachten Sensorschicht statt, wie dies vielfach bei großen Schichtdicken zu beobachten ist.

Ferner ist der Wasserstoffsensor gemäß einer weiteren Ausführungsform dadurch fortgebildet, dass das Sensormedium eine mittels Sputterdeposition hergestellte dünne Schicht ist.

Alternativ zur Sputterdeposition, wobei es sich bevorzugt um eine Herstellung mittels DC-Magnetron-Sputterdeposition handelt, können auch andere Dünnschichtverfahren eingesetzt werden, beispielsweise Aufdampfen, Laserdeposition oder dergleichen.

Gemäß einer weiteren Ausführungsform ist der Wasserstoffsensor dadurch fortgebildet, dass auf dem Sensormedium eine Deckschicht vorhanden ist. Die Deckschicht ist auf einer Oberfläche des Sensormediums vorgesehen, welche ohne Deckschicht mit der Umgebung in Kontakt steht. Die Deckschicht ist für Wasserstoff durchlässig. Beispielsweise kann die Deckschicht eine mittels CVD (Chemical Vapor Deposition) hergestellte $SiO_2$-Schicht sein. Die Deckschicht kann als Schutzschicht gegen aggressive Bestandteile der Umgebung dienen. Sie kann außerdem dazu eingesetzt werden, um die elastomechanischen Eigenschaften des Substrates und der wasserstoffabsorbierenden Schicht aus dem Sensormedium auf einander abzustimmen.

Vorzugsweise ist die Messeinheit dazu eingerichtet, einen ohmschen Widerstand des Substrats im Sensorbereich zu messen und aus dem Wert des gemessenen ohmschen Widerstands eine Wasserstoffkonzentration in der Umgebung zu bestimmen.

In einer erfindungsgemäßen Messvorrichtung wird anhand des ohmschen Widerstands von im Sensorbereich im Substrat gebildeten oder vorhandenen Widerständen oder deren Differenz, beispielsweise mit Hilfe einer Wheatstone'schen Brückenschaltung, auf die von der Sensorschicht in das Substrat eingebrachte mechanische Spannung geschlossen. Aus dem Wert der mechanischen Spannung kann auf eine Dehnung/Volumenänderung der Sensorschicht, welche die mechanischen Spannungen verursacht, geschlossen werden. Diese Volumenänderung korreliert wiederum direkt mit der Menge des von dem Sensormedium aufgenommenen Wasserstoffs. Unter der validen Annahme, dass sich die Konzentration von Wasserstoff im Sensormedium im Gleichgewicht mit der Umgebung, in der die Wasserstoffkonzentration gemessen wird, befindet, kann anhand des gemessenen ohmschen Widerstands direkt auf die in dieser Umgebung vorliegende Wasserstoffkonzentration geschlossen werden.

Gemäß einem nicht beanspruchten Verfahren zum Herstellen eines Wasserstoffsensors gemäß einer oder mehrerer der zuvor genannten Ausführungsformen, wird das Sensormedium als dünne Schicht mittels Sputterdeposition, insbesondere mittels Magnetron-Sputterdeposi-tion, auf das Substrat aufgebracht wird.

Die Herstellung der Sensorschicht mittels Sputterdeposition hat sich als sehr effizientes Verfahren herausgestellt.

Die Aufgabe wird außerdem gelöst durch ein Verfahren zum Messen einer Wasserstoffkonzentration in einer Umgebung, welches durch die folgenden Schritte fortgebildet ist:

- Exponieren eines Wasserstoff absorbierenden Sensormediums in der Umgebung, wobei das Sensormedium als dünne Schicht in einem Sensorbereich eines Substrats aufgebracht ist und das Substrat zumindest im Sensorbereich ein piezoresistiver Halbleiter ist,

- Messen eines ohmschen Widerstands des Substrats, insbesondere eines ohmschen Widerstands des Substrats im Sensorbereich,

- Bestimmen einer Wasserstoffkonzentration in der Umgebung aus dem Wert des gemessenen ohmschen Widerstands.

Auch auf das Verfahren zum Messen der Wasserstoffkonzentration treffen gleiche oder ähnliche Vorteile zu, wie sie bereits im Hinblick auf den Wasserstoffsensor selbst erwähnt wurden.

Gemäß weiterer Ausführungsformen wird als piezoresistiver Halbleiter ein Material verwendet, dessen k-Wert $\geq 2$, insbesondere $\geq 5$, ferner insbesondere $\geq 10$ und weiterhin ferner insbesondere $\geq 25$ ist. Als k-Wert wird im Kontext der vorliegenden Beschreibung der Proportionalitätsfaktor zwischen einem Quotienten aus dehnungsbedingter Längenänderung zur Ausgangslänge und einer dehnungsbedingten Widerstandsänderung zum Ausgangswiderstand auf einer Widerstandsmessstrecke des Halbleiters verstanden. Dieser Zusammenhang wird auch durch untenstehende Gleichung beschrieben:

$$\Delta R/R = k * \Delta L/L$$

In der oben genannten Gleichung ist R der ohmsche Widerstand des piezoresistiven Halbleiters im Sensorbereich, gemessen auf der Länge L. $\Delta R$ ist die Änderung des ohmschen Widerstands, welche durch die Längenänderung $\Delta L$ bedingt ist. Die Längenänderung $\Delta L$ ist eine Folge der Verformung des Substrats im Sensorbereich in Folge der in diesem Bereich durch das Sensormedium eingebrachten mechanischen Spannungen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1          eine Messvorrichtung mit einem Wasserstoffsensor in schematisch vereinfachter Querschnittsansicht, wobei das Sensormedium keiner Wasserstoffkonzentration ausgesetzt ist,

Fig. 2          diese Messvorrichtung, ebenfalls in schematisch vereinfachter Querschnittsansicht, wobei das Sensormedium einer Wasserstoffkonzentration ausgesetzt ist,

Fig. 3       eine schematisch vereinfachte Draufsicht auf einen Wasserstoffsensor,

Fig. 4       eine schematisch vereinfachte perspektivische Ansicht eines weiteren Wasserstoffsensors,

Fig. 5       ein vereinfachtes Schaltbild der Verschaltung der im Sensorbereich des piezoresistiven Halbleiters gemessenen Widerstände, die in Form einer Wheatstone'schen Brückenschaltung verschaltet sind, und

Fig. 6 bis 8       beispielhafte Messungen verschiedener Wasserstoffkonzentrationen in einer Umgebung in Abhängigkeit von der Zeit, durchgeführt mit einem Wasserstoffsensor gemäß Aspekten der Erfindung.

In den Zeichnungen sind jeweils gleiche oder gleichartige Elemente und/oder Teile mit denselben Bezugsziffern versehen, so dass von einer erneuten Vorstellung jeweils abgesehen wird.

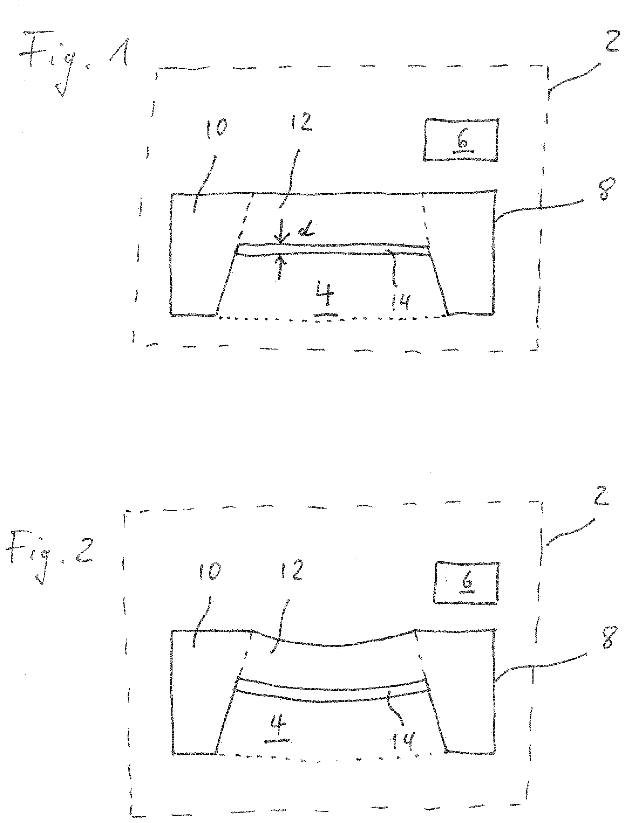

Fig. 1 zeigt in schematisch vereinfachter Querschnittsansicht eine Messvorrichtung 2 zur Messung einer Wasserstoffkonzentration in einer Umgebung 4. Die Umgebung 4 steht beispielsweise über Kanäle, Anschlüsse oder dergleichen in Kontakt mit einer weiteren Umgebung oder Atmosphäre, in der eine Wasserstoffkonzentration gemessen werden soll. Hierzu werden geeignete Maßnahmen ergriffen, um in der Messvorrichtung 2 die Umgebung 4 abzuschließen und über Anschlüsse mit dieser weiteren Messumgebung zu koppeln. Die Messvorrichtung 2 umfasst ferner eine Messeinheit 6, bei der es sich beispielsweise um einen Computer, einen Mikrokontroller oder auch um ein in weiterer Einheit implementiertes geeignetes Softwaremittel handelt. Die Messeinheit ist dazu eingerichtet einen ohmschen Widerstand zu messen und umfasst hierzu beispielsweise unter anderem eine Spannungsquelle und ein Spannungsmessgerät. Die Messvorrichtung 2 umfasst ferner einen Wasserstoffsensor 8, der zur Messung der Wasserstoffkonzentration in der Umgebung 4 eingerichtet ist. Der Wasserstoffsensor 8 umfasst ein Substrat 10, auf dem in einem mit der Umgebung 4 kommunizierenden Sensorbereich 12 ein wasserstoffabsorbierendes Sensormedium 14 als dünne Schicht aufgebracht ist.

Lediglich beispielhaft ist die Umgebung 4 auf einer Unterseite des Sensorbereichs 12 angeordnet. Es ist ebenso vorgesehen, dass das Sensormedium 14 als dünne Schicht auf einer Oberseite des Sensorbereichs 12 aufgebracht wird und sich dementsprechend die Umgebung 4, in der die Wasserstoffkonzentration gemessen wird, auf der Oberseite befindet. Zwingend ist jedoch vorgesehen, dass sich die Sensorschicht 14 auf nur einer Seite des beispielsweise als Si-Membran ausgestalteten Sensorbereichs 12 befindet und diese Seite, d.h. die Sensorschicht mit der wasserstoffhaltigen Umgebung 4 kommuniziert.

Das Sensormedium 14 ist beispielsweise ein Metall oder eine Metalllegierung. Beispielsweise handelt es sich bei dem Sensormedium 14 um eine dünne Schicht aus Palladium, Yttrium, Scandium, einem Lanthanid, einem Actionid, Wolframoxid oder Vanadiumoxid, wobei auch Legierungen und Mischungen dieser Materialien vorgesehen sind. Insbesondere ist vorgesehen, dass das Sensormedium 14 eine Legierung aus Palladium und Gold (PdAu) oder aus Palladium und Nickel (PdNi) ist, welche durch Co-Sputterdeposition hergestellt ist. Hierzu wird das Substrat 10 in den Rezipienten einer Sputteranlage eingebracht und es wird die PdAu- oder PdNi-Schicht direkt auf das Substrat 10 aufgebracht.

Die Schichtdicke d des als dünne Schicht auf das Substrat 10 aufgebrachten Sensormediums 14 ist beispielsweise geringer als 500 nm, sie liegt ferner beispielsweise zwischen 5 nm und 100 nm und ferner beispielsweise zwischen 5 nm und 20 nm.

Das Sensormedium 14 ist ein Material, welches sein Volumen in Abhängigkeit einer in dem Sensormedium 14 vorhandenen Wasserstoffkonzentration verändert. Diese Variation des Volumens verändert die von dem Sensormedium 14 in das Substrat 10 im Sensorbereich 12 eingebrachte mechanische Spannung. Diesen Effekt zeigt ein Vergleich zwischen den Fig. 1 und 2.

Fig. 2 zeigt die Messvorrichtung 2, ebenfalls in schematisch vereinfachter Querschnittansicht, wobei das Sensormedium 14 nun einer in der Umgebung 4 vorhandenen Wasserstoffkonzentration ausgesetzt ist. Dies bedeutet, dass das Sensormedium 14 sich ausdehnt und diese Ausdehnung zu der schematisch und aus Gründen der Deutlichkeit übermäßig stark dargestellten Verbiegung des Substrats 10 im Sensorbereich 12 führt. Befindet sich das Sensormedium 14 auf der gegenüberliegenden oberen Seite des Substrats 10, wird sich dieses in die entgegengesetzte Richtung verbiegen. Das Substrat 10 ist zumindest im Sensorbereich 12 ein piezoresistiver Halbleiter. Die in dem Sensormedium 14 entstehenden mechanischen Spannungen führen dazu, dass auch das Substrat 10 zumindest im Sensorbereich 12 mechanischen Spannungen ausgesetzt wird. Diese mechanischen Spannungen können unter Ausnutzung des piezoresistiven Effekts von der Messeinheit 6 erfasst werden. Hierzu ist die Messeinheit 6 über in den Figuren nicht dargestellte elektrische Verbindungen mit dem Substrat 10 kontaktiert.

Die Messeinheit 6 misst den ohmschen Widerstand bzw. eine Veränderung des ohmschen Widerstands des Substrats 10 zumindest im Sensorbereich 12. Aus dem Wert des gemessenen ohmschen Widerstands kann auf eine Wasserstoffkonzentration in der Umgebung 4 geschlossen werden.

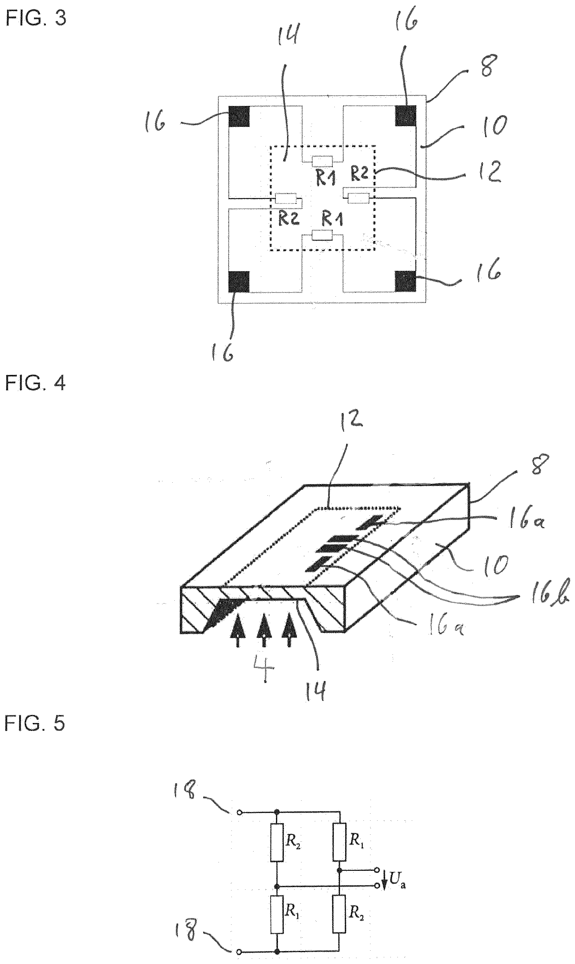

Fig. 3 zeigt in schematisch vereinfachter Draufsicht einen Wasserstoffsensor 8. Beispielhaft soll in Fig.

3 eine Draufsicht auf diejenige Seite eines Substrats 10 des Wasserstoffsensors 8 gezeigt sein, auf welcher in dem Sensorbereich 12 das Sensormedium 14 aufgebracht ist. Das Substrat 10 umfasst Kontaktpads 16, über die der Wasserstoffsensor 8 mit der Messeinheit 6 kontaktiert ist. Über die Kontaktpads 16 ist es möglich, Veränderungen der Widerstände R1, R2, die in Form einer Wheatstone'schen Brückenschaltung verschaltet sind, zu bestimmen.

Fig. 4 zeigt eine schematisch vereinfachte perspektivische Ansicht eines weiteren Wasserstoffsensors 8. Dieser umfasst in einem Sensorbereich 12 ein von der Unterseite her aufgebrachtes Sensormedium 14, welches, wie mit Pfeilen angedeutet, einer wasserstoffhaltigen Umgebung 4 ausgesetzt wird. Die durch das Sensormedium 14 bedingten mechanischen Spannungen werden mithilfe von Widerstandsmessungen erfasst, wobei Abgriffe an den Kontaktpads 16 erfolgen, mit denen ein ohmscher Widerstand des Substrats 10 im Sensorbereich 12 gemessen werden kann. Erneut kann beispielsweise eine Verschaltung in Form einer Wheatstone'schen Brückenschaltung erfolgen. Dabei wird der Widerstand zwischen den Kontaktpads 16a als Widerstand R2 und der Widerstand zwischen den Kontaktpads 16b als Widerstand R1 gemessen.

Fig. 5 zeigt ein schematisch vereinfachtes Schaltbild der Verschaltung der beispielhaft im Sensorbereich 12 des piezoresistiven Halbleiters gemessenen ohmschen Widerstände R1 und R2, die in Form einer Wheatstone'schen Brückenschaltung verschaltet sind. Es wird an die beiden Klemmen 18 eine Spannung angelegt und es wird die Diagonalspannung oder Brückenspannung Ua, der als Spannungsteiler verschalteten Widerstände R1, R2 gemessen. Eine Veränderung der Brückenspannung Ua ist ein Maß für die Veränderung der Widerstände R1, R2 und damit ein direktes Maß für die von dem Sensormedium 14 in das Substrat 10 im Sensorbereich 12 eingetragenen mechanischen Spannungen. Somit kann direkt an der Brückenspannung Ua die Wasserstoffkonzentration in der Umgebung 4 abgelesen werden.

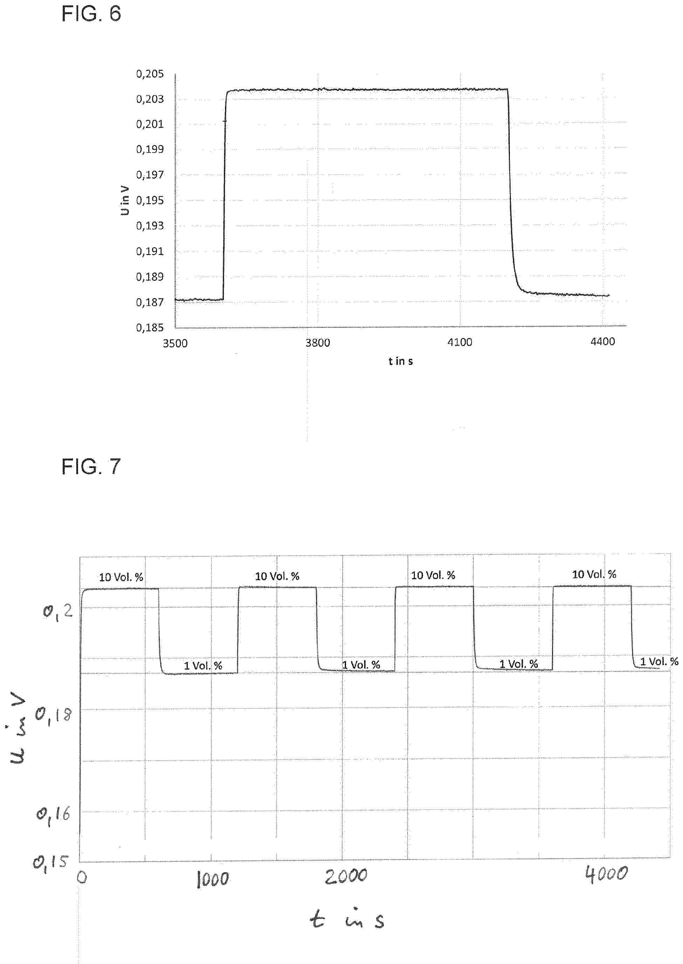

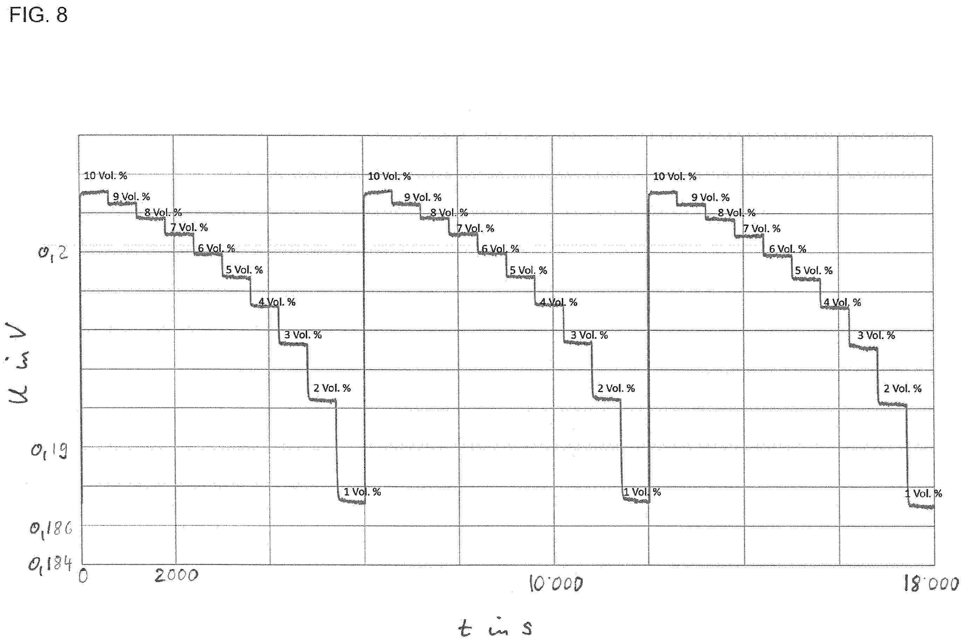

Fig. 6 bis 8 zeigen beispielhafte Messungen verschiedener Wasserstoffkonzentrationen in der Umgebung 4 in Abhängigkeit von der Zeit t. Auf der vertikalen Achse ist die elektrische Spannung in Volt angegeben, während die horizontale Achse die Zeit t in Sekunden zeigt. Die auf der vertikalen Achse dargestellte elektrische Spannung ist ein direktes Maß für die im Sensorbereich 12 herrschende mechanische Spannung und somit ebenfalls ein direktes Maß für die Wasserstoffkonzentration in der Umgebung 4. Es ist deutlich sichtbar, dass der Wasserstoffsensor 8 ein sehr konstantes Messergebnis in einem großen Messbereich liefert. In Fig. 6 startet die Messung bei einer Konzentration von 1vol% Wasserstoff in Luft und steigt auf 10vol% Wasserstoff an. In der in Fig. 7 gezeigten Messung werden ebenfalls Konzentrationen von 1vol% Wasserstoff in Luft und 10vol% gemessen. Die Messungen in Fig. 6 und 7 wurden jeweils für eine maximale Dauer von 4400 s durchgeführt, was 73 min entspricht. Fig. 8 zeigt eine weitere Messung, bei der Wasserstoffkonzentrationen zwischen 1vol% und 10vol% gemessen wurden. Der Wasserstoffsensor 8 zeigt eine hohe Sensitivität, ein schnelles Ansprechverhalten (Fig. 6), einen zeitlich stabilen Messwert (Fig. 6) und in hohem Maße reproduzierbare Messergebnisse (Fig. 7 und 8).

Bezugszeichenliste

| 2 | Messvorrichtung |
|---|---|
| 4 | Umgebung |
| 6 | Messeinheit |
| 8 | Wasserstoffsensor |
| 10 | Substrat |
| 12 | Sensorbereich |
| 14 | Sensormedium |
| 16, 16a, 16b | Kontaktpads |
| 18 | Klemme |
| | |
| d | Schichtdicke |
| R1, R2 | Widerstände |
| Ua | Brückenspannung |

**Patentansprüche**

1.  Messvorrichtung (2) zur Messung einer Wasserstoffkonzentration in einer Umgebung ( 4 ), umfassend eine Messeinheit (6) und einen Wasserstoffsensor (8) zur Messung einer Wasserstoffkonzentration in einer Umgebung ( 4 ), wobei der Wasserstoffsensor (8) ein Substrat (10) umfasst, auf dem in einem mit der Umgebung kommunizierenden Sensorbereich (12) ein Wasserstoff absorbierendes Sensormedium (14) als dünne Schicht aufgebracht ist, wobei das Sensormedium (14) sein Volumen in Abhängigkeit einer in dem Sensormedium (14) vorhandenen Wasserstoffkonzentration verändert, und diese Variation des Volumens eine Variation einer von dem Sensormedium (14) in das Substrat (10) eingebrachten mechanischen Spannung bewirkt, wobei das Substrat (10) zumindest im Sensorbereich (12) ein piezoresistiver Halbleiter ist, wobei das Sensormedium (14) mit der Umgebung (4) kommuniziert, **dadurch gekennzeichnet, dass** die Messeinheit (6) dazu eingerichtet ist, einen ohmschen Widerstand des Substrats (10) zu messen und aus dem Wert des gemessenen ohmschen Widerstands eine Wasserstoffkonzentration in der Umgebung (4) zu bestimmen.

2.  Messvorrichtung (2) nach Anspruch 1, **dadurch gekennzeichnet, dass** das Sensormedium (14) ein Metall oder eine Metalllegierung ist.

**3.** Messvorrichtung (2) nach Anspruch 1 oder 2, **dadurch gekennzeichnet, dass** das Sensormedium (14) aus Palladium, Yttrium, Scandium, einem Lanthanid, einem Actionid, Wolframoxid und/oder Vanadiumoxid ist.

**4.** Messvorrichtung (2) nach Anspruch 1 oder 2, **dadurch gekennzeichnet, dass** das Sensormedium (14) eine Palladiumlegierung oder eine Mischung, Legierung oder Verbindung aus mehreren der folgenden Materialien ist: Palladium, Yttrium, Scandium, einem Lanthanid, einem Actionid, Wolframoxid, Vanadiumoxid.

**5.** Messvorrichtung (2) nach einem der Ansprüche 1 bis 4, **dadurch gekennzeichnet, dass** das Sensormedium (14) eine Legierung aus Palladium und Gold ($Pd_xAu_y$-Legierung) oder eine Legirung aus Palladium und Nickel ($Pd_xNi_y$-Legierung ist, wobei insbesondere der Anteil von Gold oder Nickel zwischen 0,5at% und 50at% liegt ($Pd_{99,5}Au_{0,5}$-Legierung bis $Pd_{50}Au_{50}$-Legierung oder $Pd_{99,5}Ni_{0,5}$-Legierung bis $Pd_{50}Ni_{50}$-Legierung)

**6.** Messvorrichtung (2) nach einem der Ansprüche 1 bis 5, **dadurch gekennzeichnet, dass** das als dünne Schicht ausgebildete Sensormedium (14) eine Schichtdicke (d) aufweist, die geringer als 500 nm ist, die insbesondere zwischen 5 nm und 100 nm und ferner insbesondere zwischen 5 nm und 20 nm liegt.

**7.** Messvorrichtung (2) nach einem der Ansprüche 1 bis 6, **dadurch gekennzeichnet, dass** das Sensormedium (14) eine mittels Sputtereposition hergestellte dünne Schicht ist.

**8.** Messvorrichtung (2) nach einem der Ansprüche 1 bis 7, wobei die Messeinheit (6) dazu eingerichtet ist, einen ohmschen Widerstand des Substrats (10) im Sensorbereich (12) zu messen und aus dem Wert des gemessenen ohmschen Widerstands eine Wasserstoffkonzentration in der Umgebung (4) zu bestimmen.

**9.** Verfahren zum Messen einer Wasserstoffkonzentration in einer Umgebung (4), **gekennzeichnet durch** die folgenden Schritte:

- Exponieren eines Wasserstoff absorbierenden Sensormediums (14) in der Umgebung (4), wobei das Sensormedium (14) als dünne Schicht in einem Sensorbereich (12) eines Substrats (10) aufgebracht ist und das Substrat (10) zumindest im Sensorbereich (12) ein piezoresistiver Halbleiter ist,
- Messen eines ohmschen Widerstands des Substrats (10), insbesondere eines ohmschen Widerstands des Substrats (10) im Sensorbereich (12),
- Bestimmen einer Wasserstoffkonzentration in der Umgebung (4) aus dem Wert des gemessenen ohmschen Widerstands.

**Claims**

**1.** A measuring device (2) for measuring a hydrogen concentration in an environment (4), comprising a measuring unit (6) and a hydrogen sensor (8) for measuring a hydrogen concentration in an environment (4), wherein the hydrogen sensor (8) comprises a substrate (10), on which a hydrogen-absorbing sensor medium (14) is applied as a thin film in a sensor region (12) communicating with the environment, wherein the sensor medium (14) changes its volume depending on a hydrogen concentration in the sensor medium (14), and said change of the volume causes a variation of a mechanical strain introduced by the sensor medium (14) in the substrate (10), wherein the substrate (10) is a piezoresistive semiconductor, at least within the sensor region (12), wherein the sensor medium (14) communicates with the environment (4), **characterized in that** the measuring unit (6) is configured to measure an ohmic resistance of the substrate (10) and to determine a hydrogen concentration in the environment (4) from the value of the measured ohmic resistance.

**2.** The measuring device (2) according to claim 1, **characterized in that** the sensor medium (14) is a metal or a metal alloy.

**3.** The measuring device (2) according to claim 1 or 2, **characterized in that** the sensor medium (14) comprises palladium, yttrium, scandium, a lanthanide, an actinide, tungsten oxide and/or vanadium oxide.

**4.** The measuring device (2) according to claim 1 or 2, **characterized in that** the sensor medium (14) is a palladium alloy, or a mixture, alloy or compound consisting of a plurality of the following materials: palladium, yttrium, scandium, a lanthanide, an actinide, tungsten oxide, and vanadium oxide.

**5.** The measuring device (2) according to one of claims 1 to 4, **characterized in that** the sensor medium (14) is an alloy consisting of palladium and gold ($Pd_x$-$Au_y$ alloy), or an alloy consisting of palladium and nickel ($Pd_x$Niy alloy), wherein in particular the portion of gold or nickel lies between 0.5 at% and 50 at% ($Pd_{0,5}Au_{99,5}$ alloy to $Pd_{50}Au_{50}$ alloy, or $Pd_{0,5}Ni_{99,5}$ alloy to $Pd_{50}Ni_{50}$ alloy)

**6.** The measuring device (2) according to one of claims 1 to 5, **characterized in that** the sensor medium (14) is a thin film having a film thickness (d) that is less than 500 nm and in particular the film thickness (d) is between 5 nm and 100 nm, and further in particular the film thickness (d) is between 5 nm and 20 nm.

**7.** The measuring device (2) according to one of claims 1 to 6, **characterized in that** the sensor medium (14) is a thin film made by sputter deposition.

**8.** The measuring device (2) according to one of claims 1 to 7, wherein the measuring unit (6) is configured to measure an ohmic resistance of the substrate (10) within the sensor region (12), and to determine a hydrogen concentration in the environment (4) from the value of the measured ohmic resistance.

**9.** A method for measuring a hydrogen concentration in an environment (4), **characterized by** the following steps:

- exposing a hydrogen-absorbing sensor medium (14) to the environment (4), wherein the sensor medium (14) is applied as a thin film in a sensor region (12) of a substrate (10), and the substrate (10) is a piezoresistive semiconductor, at least within the sensor region (12),
- measuring an ohmic resistance of the substrate (10), in particular an ohmic resistance of the substrate (10) within the sensor region (12),
- determining a hydrogen concentration in the environment (4) from the value of the measured ohmic resistance.

**Revendications**

**1.** Dispositif de mesure (2) pour mesurer une concentration d'hydrogène dans un environnement (4), comprenant une unité de mesure (6) et un capteur d'hydrogène (8) pour mesurer une concentration d'hydrogène dans un environnement (4), le capteur d'hydrogène (8) comprenant un substrat (10) sur lequel un milieu de détection (14) absorbant l'hydrogène est appliqué sous forme de couche mince dans une zone de détection (12) communiquant avec l'environnement, le milieu de détection (14) faisant varier son volume en fonction d'une concentration d'hydrogène présente dans le milieu de détection (14), et cette variation de volume provoque une variation d'une tension mécanique introduite par le milieu de détection (14) dans le substrat (10), le substrat (10) étant, au moins dans la zone de détection (12), un semi-conducteur piézo-résistant, le milieu de détection (14) communiquant avec l'environnement (4), **caractérisé en ce que** l'unité de mesure (6) est conçue de façon à mesurer une résistance ohmique du substrat (10) et pour déterminer une concentration d'hydrogène dans l'environnement (4) à partir de la valeur de la résistance ohmique mesurée.

**2.** Dispositif de mesure (2) selon la revendication 1, **caractérisé en ce que** le milieu de détection (14) est un métal ou un alliage de métaux.

**3.** Dispositif de mesure (2) selon la revendication 1 ou la revendication 2, **caractérisé en ce que** le milieu de détection (14) est constitué de palladium, d'yttrium, de scandium, d'un lanthanide, d'un actionide, d'oxyde de tungstène et/ou d'oxyde de vanadium.

**4.** Dispositif de mesure (2) selon la revendication 1 ou la revendication 2, **caractérisé en ce que** le milieu de détection (14) est un alliage de palladium ou un mélange, un alliage ou un composé de plusieurs des matériaux suivants : palladium, yttrium, scandium, un lanthanide, un actionide, de l'oxyde de tungstène, de l'oxyde de vanadium.

**5.** Dispositif de mesure (2) selon l'une des revendications 1 à 4, **caractérisé en ce que** le milieu de détection (14) est un alliage de palladium et d'or (alliage $Pd_xAu_y$) ou un alliage de palladium et de nickel (alliage $Pd_xNi_y$), la proportion d'or ou de nickel étant notamment comprise entre 0,5 at% et 50 at% (alliage $Pd_{99,5}Au_{0,5}$ à alliage $Pd_{50}Au_{50}$ ou alliage $Pd_{99,5}Ni_{0,5}$ à alliage $Pd_{50}Ni_{50}$).

**6.** Dispositif de mesure (2) selon l'une des revendications 1 à 5, **caractérisé en ce que** le milieu de détection (14) réalisé sous forme de couche mince présente une épaisseur de couche (d) qui est inférieure à 500 nm, qui est notamment comprise entre 5 nm et 100 nm et en outre notamment comprise entre 5 nm et 20 nm.

**7.** Dispositif de mesure (2) selon l'une des revendications 1 à 6, **caractérisé en ce que** le milieu de détection (14) est une couche mince obtenue par pulvérisation cathodique.

**8.** Dispositif de mesure (2) selon l'une des revendications 1 à 7, dans lequel l'unité de mesure (6) est agencée de façon à mesurer une résistance ohmique du substrat (10) dans la zone de détection (12) et pour déterminer une concentration en hydrogène dans l'environnement (4) à partir de la valeur de la résistance ohmique mesurée.

**9.** Procédé de mesure d'une concentration d'hydrogène dans un environnement (4), **caractérisé par** les étapes suivantes :

- exposition d'un milieu de détection (14) absorbant l'hydrogène dans l'environnement (4), le milieu de détection (14) étant appliqué sous forme d'une couche mince dans une zone de détection (12) d'un substrat (10) et le substrat (10) étant un semi-conducteur piézo-résistant au moins dans la zone de détection (12),
- mesure d'une résistance ohmique du substrat (10), en particulier d'une résistance ohmique du substrat (10) dans la zone de détection (12),
- détermination d'une concentration d'hydrogène dans l'environnement (4) à partir de la valeur de la résistance ohmique mesurée.

Fig. 1

2

10    12

6

8

α

4

14

Fig. 2

2

10    12

6

8

4

14

FIG. 3

FIG. 4

FIG. 5

FIG. 6

FIG. 7

FIG. 8

**IN DER BESCHREIBUNG AUFGEFÜHRTE DOKUMENTE**

**In der Beschreibung aufgeführte Patentdokumente**

- DE 102006018767 A1
- US 7791150 B1
- KR 20180074100
- CN 108872314

**In der Beschreibung aufgeführte Nicht-Patentliteratur**

- **HONGCHUAN JIANG et al.** *Low Concentration Response Hydrogen Sensors Based on Wheatstone Bridge,* 2019